# EUROPEAN PATENT APPLICATION

(11) **EP 1 426 076 A2**
(43) Date of publication of application: **09.06.2004**
(21) Application number: 03029791.5
(22) Date of filing: 24.01.2001
(51) Int. Cl.: A61M 31/00, A61K 9/00, A61L 27/40

(54) **Medical device**

(30) Priority: 04.02.2000 SE 0000363
(62) Divisional of application: 01902896.8
(71) Applicant: Zoucas Kirurgkonsult AB, 21773 Malmö (SE)
(72) Inventor: Harnek, Jan, 217 73 Malmö (SE); Zouka, Eftichia-Vassiliki, 217 73 Malmö (SE)
(74) Representative: Wiklund, Erik

(57) **Abstract**

There is provided a medical device chosen from sutures, surgical staples, implants and catheters adapted for insertion into a human or animal body, characterised in that its exterior surface is coated with
*i)* an inner first layer of a biocompatible carrier providing sustained release of a biologically active agent dissolved or dispersed therein;
*ii)* an outer second layer consisting of a film of said biologically active agent applied on said inner first layer, where said film optionally may contain at least one non-polymeric adjuvant, diluent or carrier.

The present medical device is especially well suited for treatment or prevention of restenosis and disorders related thereto.

## Description

### Field of the Invention

The present invention relates to a medical device chosen from sutures, surgical staples, implants and catheters adapted for insertion into a human or animal body as well as a method for use thereof in promoting tissue healing and in treatment of restenosis and disorders related thereto.

### Background of the Invention

During the last years, local drug administration has become an increasingly more attractive means of treatment of various disorders. As is well known, local drug administration mainly offers both a reduced risk of unwanted systemic side-effects and much less general inconvenience for all parties involved. Hence, a vast number of various medical devices and methods providing direct application of drug(s) to a diseased site have been disclosed. Typical such medical devices and methods are disclosed in US 5 861 168, WO 96/35416 and WO 99/08729, the teachings of which are incorporated herein by reference.

Stenotic lesions of vasculature are common disorders which often lead to arterial occlusive disease. Indeed, the latter is the most frequently encountered problem of vascular disease, and particularly of cardiovascular disease. In general, approximately 50% of the patients with significant cardiovascular disease will be treated with percutaneous coronary angioplasty, whereby a balloon angioplasty is usually performed. However, the high incidence of restenosis, reaching 30-50% in several studies, following such ballon angioplasty continues to restrict the long-term success of this procedure (Kastrati, A., Schomig, A., Elezi, S., Schulen, H., Wilhelm, M., Dirschinger, J., *Circ*., 97, 2396 (1998)).

In order to treat the aforementioned resulting restenosis, stent implantation has lead to some success.

Various medical devices having a coating which provides local rapid release of nitric oxide (NO) have provided a potentially more successful alternative. Typical such medical devices are disclosed in WO 96/35416 referred to above. This reference suggests many types of medical devices providing release of NO, such as *i)* a medical device partially or completely coated with a nitric oxide adduct either as the coating per se or in a coating matrix, *ii)* a medical device partially or completely produced from a material which includes an NO adduct, and *iii)* a medical device derivatised with an NO adduct. As for coated stents, WO 96/35416 explicitly discloses only a Palmaz-Schatz stent coated with a layer of a bovine serum albumine (BSA) conjugate of S-nitrosothiol (Example 5). All the other examples relate to coated catheters. Related teachings are disclosed in WO 99/08729, where a balloon catheter coated with a layer of molsidomine is utilised. The characterising features of the medical device according to the present invention are neither disclosed nor suggested in any one of these references.

Here it should be mentioned that molsidomine is a recently introduced nitric oxide donor which belongs to the substance group of sydnonimines. This type of compounds are known for their ability to release NO without need of enzymatic catalysis (Lablanche, J-M. *et al., Circ*., 95(1), 83 (1997)). Diethylenetriamine/nitric oxide adduct (DETA/NO) is a similar NO releasing compound. (Maragos C.M. et al. J. Med Chem. 34:3242-3247.(1991)

As for the aforementioned types of coated medical devices, two main problems are associated therewith. Firstly, the type of coating used is not potent enough to promote tissue healing, particularly vascular healing, to such an extent that beneficial long-term effects are attained. Accordingly, the hitherto known coatings are not potent enough to treat restenosis in such a manner that it ceases to be detrimental to the patient on a more long-term basis. Secondly, said type of coating elicits virtually no prophylactic effect. There is of course a strong demand in the art to provide a medical device which does not suffer from these disadvantages.

### Disclosure of the Invention

According to the present invention, there is provided a novel medical device chosen from sutures, surgical staples, implants and catheters which inter alia overcomes the problems referred to above. Indeed, the features of the present medical device provide a solution of these problems also for many other types of disorders (*vide infra*) in addition to vascular damage(s) and restenosis. More specifically, the present invention relates to a medical device chosen from sutures, surgical staples, implants and catheters adapted for insertion into a human or animal body, characterised in that its exterior surface is coated with
*i)* an inner first layer of a biocompatible carrier providing sustained release of a biologically active agent dissolved or dispersed therein;
*ii)* an outer second layer consisting of a film of said biologically active agent applied on said inner first layer, where said film optionally may contain at least one non-polymeric adjuvant, diluent or carrier.

The expression "biologically active agent", as used herein, comprises any substance(s) which may yield a physiological response when administered to a living organism. Thus, said biologically active agent may also be an active metabolite, drug progenitor or a drug-conjugate, such as a drug-protein (*e.g.* drug-BSA) conjugate or a drug-spacer conjugate, where the protein or spacer is selected in such a manner that it will readily adhere to said inner first layer, *i.e.* to said biocompatible carrier. The conjugates may be formed by either covalent binding or other sufficiently strong intermolecular binding resulting from e.g. hydrophobic, hydrogen-binding or hydrophilic interactions.

It should be understood that said biologically active agent may also be a mixture of one or more physiologically active substances, which are used in a particular combination. In this case, the combination is present in both said first and second layer, albeit not necessarily in the same concentration and/or ratio.

The expression "sustained release", as used herein, means that said biocompatible carrier releases no more than 50-90 percent by weight (wt%) of said biologically active agent dissolved or dispersed therein within 7 days after insertion of said medical device into a human or animal body.

Typically, said biocompatible carrier is a polymer. It is preferably selected from polyamine-dextran-sulphate, poly fatty acid esters, polyurethane and other pharmaceutically acceptable polymeric carriers known in the art. Thus, the following polymers can provide a suitable biocompatible carrier according to the present invention: poly fatty acid esters [*e*.*g*. homopolymer (*e*.*g*. polylactic acid) of fatty acid or copolymer (*e*.*g*. copolymer of lactic acid/glycolic acid, copolymer of 2-hydroxy butyric acid/glycolic acid) of two or more fatty acids, a mixture of the homopolymer and/or copolymer (*e.g*. a mixture of polylactic acid and copolymer of 2-hydroxybutyric acid/glycolic acid), examples of the fatty acid include α-hydroxycarboxylic acid (*e*.*g*. glycolic acid, lactic acid, 2-hydroxy butyric acid, 2-hydroxy-valeric acid, 2-hydroxy-3-methyl butyric acid, 2-hydroxy-caproic acid, 2-hydroxyisocaproic acid, 2-hydroxycaprylic acid), cyclic dimers of α-hydroxycarboxylic acids (*e*.*g*. glycolide, lactide), hydroxydicarboxylic acid (*e.g.* malic acid), hydroxytricarboxylic acid (*e.g.* citric acid)], poly-α-cyanoacrylate, polyalkylene oxalates (*e*.*g*. polytrimethylene oxalate, polytetramethylene oxalate), poly ortho esters, poly ortho carbonates and other polycarbonates (*e.g*. polyethylene carbonate, poly-ethylenepropylene carbonate), polyamino acids (*e.g*. poly-γ-benzyl-L-glutamic acid, poly-L-alanine, poly-γ-methyl-L-glutamic acid), polylysine, and the like. Further examples of a suitable biocompatible carrier include polyacrylic acid, polymethacrylic acid, copolymer of acrylic acid and methacrylic acid, polyethyle glycol, silicon polymer, dextran stearate, ethylcellulose, acetylcellulose, maleic anhydride copolymers, ethylene-vinylacetate copolymer, polyvinyl acetate, polyvinyl alcohol, polyacrylamide and the like. These polymers may be used alone or in combination. They may be used in the form of a copolymer or mere mixture of these two or more polymers. They may be in the form of salts thereof. The affinity for the adsorbed molecular coating e.g. film can be enhanced by attachment of phenylboronic acid moities. For the purposes of the present invention, D-, L- and D,L-isomers are equally suitable.

Preferably, said non-polymeric adjuvant, diluent or carrier is selected from phosphorylcholine and derivatised phosphorylcholine, albumines, liposomes, and contrast medium; preferably iohexole.

As a non-limiting example of suitable derivatised phosphorylcholines, mention can be made of the compounds disclosed in WO 91/13639 and WO 93/22320, the entire teachings of which are incorporated herein by reference.

Moreover, it is preferred that said polyamine-dextran-sulphate, poly fatty acid ester and polyurethane have an average molecular weight in the range of from 5 kDa to 100 kDa.

A preferred polyamine-dextran-sulphate is that provided by Corline Systems AB (Sweden) and used as coating in the stent Joflex Heparin® This carrier material is known to be easily modifiable so that the desired rate of sustained release of a biologically active agent dissolved or dispersed therein is attained. Indeed, use of polyamine-dextran-sulphate as carrier provides a particularly efficient embodiment for slow intramural delivery of numerous different biologically active agents.

Preferably, said poly fatty acid ester is polylactic acid (PLA), polyglycolic acid (PGA) or a copolymer of lactic acid and glycolic acid (PLGA). PLGA is particularly preferred, as it is also commercially available in many varieties (inter alia provided by Boehringer Ingelheim, DE). Other preferred polymers are poly-α-cyanoacrylate and a copolymer of 2-hydroxybutyric acid and glycolic acid.

When PLGA is used, its monomer ratio is preferably about 100/0 to 50/50 (w/w). When a copolymer of 2-hydroxybutyric acid and glycolic acid is used, its monomer ratio is preferably about 100/0 to 25/75 (w/w).

The average molecular weight of PLGA and the copolymer of 2-hydroxybutyric acid and glycolic acid is preferably about 5 to 30 kDa. When a mixture of a polylactic acid (A) and a copolymer of 2-hydroxybutyric acid/glycolic acid (B) is used, the mixture can be used in a blend (w/w) ratio of about 10/90 to 90/10, preferably about 25/75 to 75/25.

The weight-average molecular weight of the polyactic acid (A) is preferably about 5 to 30 kDa.

The preferred proportion of glycolic acid in the copolymer (B) is about 40-70 mol%. The average molecular weight of the copolymer (B) is preferably about 5 to 25 kDa.

If desired, said biocompatible carrier may additionally contain other substances which are generally used in the preparation of pharmaceutical compositions. Typical such substances are pharmaceutically acceptable adjuvants, adhesives, stabilisers (often antioxidants), lubricants and pH regulators. All of these substances are well known in the art.

Said biologically active agent is preferably present in said inner first layer at a concentration of from 0.01 to 99 wt%. Preferably, said inner first layer has a thickness in the range of from 0,5 to 1000 µm.

In the present medical device, said biologically active agent is preferably an antiinflammatory drug, e.g. prostaglandines, indomethacin, or diclofenac. It is further preferred that said compound is a diethylene-triamine/nitric oxide adduct (DETA/NO) or a sydnonimine, preferably molsidomine or linsidomine.

One or more agents, i.e. adjuvants, which enhance the amount of NO delivered to the cells at the site to be treated can also be present. Such agents typically enhance the absorption of NO or its precursor, increase the activity of the NO-releasing compound and/or protect the NO-releasing compound from degradation. Particularly useful such agents are the vitamins B₆, B₁₂, C and E. Also useful in the practising of the present invention are folates, β-carotene, glutathione, coenzyme Q, cysteine, tocopherols, phenolic compounds, thiols, ubiquinones, heparinoids, Ca²⁺-antagonists, nitrates, protein kinase inhibitors, anti-thrombin and antiproliferative agents, such as methotrexate, mitomycin C, doxyrubicin, cytostatics, somatostatin analogs, cytochalasin B and dexomethasone.

In the present medical device, said exterior surface preferably consists of metal or a biocompatible organic or inorganic polymer. Said metal is preferably selected from gold, silver, platinum, stainless steel, titanium and biocompatible alloys thereof. Said biocompatible organic or inorganic polymer is preferably selected from fibrin, polytetrafluoroethylene (PTFE), silicone, silicone rubber, nylon and polyethylene perthalate (Dacron).

In the most preferred embodiment of the present invention, said medical device is a stent. In addition, the present invention relates to a method for use of said medical device as set forth above. More specifically, the present invention further relates to a method for promoting tissue healing in a human or animal body, wherein said method comprises insertion of a medical device as set forth above into a site where tissue healing is required. Even more specifically, the present invention also relates to a method for treatment or prevention of restenosis and disorders related thereto in a human or animal body, wherein said method comprises insertion of a medical device as set forth above into a site where treatment or prevention of restenosis and disorders related thereto is required.

Said site is typically an arteryor a part of the gastrointestinal tract.

The above method is also applicable to the treatment or prevention of other disorders, such as inflammatory conditions or proliferative disorders, e.g. cancer diseases. A person skilled in the art will readily realise how to adapt, if necessary, the practising of the present method to the particular disorder and circumstances at hand.

As for the typical dosage of the biologically active agent, it varies within a wide range and depends on various factors, such as the particular requirements of each receiving individual and the particular medical device used. The required dosage range depends on the used agent and circumstance under which it is applied. The dosage is generally within the range of 0.001-100 mg/kg body weight, albeit also other ranges may be required under certain circumstances.

The present invention is further illustrated by the following non-limiting general examples.

### Examples

Coating of a stent having a smooth stainless steel surface:

A stent made in electropolished Stainless steel 316L is coated by dipping it at room temperature into a paste of polyamine-dextrane-sulphate incorporated with heparine as the carrier of 3-morpholino-sydnonimine present in a concentration of 10⁻⁴ M. Concentrations as low as 10⁻⁸ M are likely also effective (in the literature a concentration of 1 nM has been reported to have an effect in vitro). The polyamine-dextrane-sulphate (PDS) is then allowed to harden by conventional means, e.g. by evaporation of a fluidizing solvent, such as ethanol, at room temperature, whereby a first layer is formed. This layer is sufficiently elastic to retain its structural integrity when the stent is subsequently expanded after insertion thereof into e.g. an artery. The outer second layer is then applied by dipping at 37°C the PDS-coated stent into a paste of polymerized phosphorylcholine containing 3-morpholino-sydnonimine (in the range from about 10⁻⁸ M to 10⁻², preferably about 10⁻⁴ M) and a fluidising solvent, such as a water/ethanol mixture. After removal of the fluidising solvent by air drying at room temperature as above, a stent having two separate drug-containing layers is provided. Alternatively, the stent is dipped several times in molsidonimine in a high concentration for approximately half an hour in a way that a film is created with or without the use of a carrier.

In said pastes, a concentration of 3-morpholino-sydnonimine of from 10⁻⁸ to 10⁻² M is usually suitable. As an alternative, the outer second layer may be applied by first coating the inner first layer with polymerized phosphorylcholine only, followed by drying the product and then dipping it into a solution of e.g. molsidomine in CHCl₃ and/or ethanol. In this manner, molsidomine is incorporated onto the layer of phosphorylcholine. A similar procedure is disclosed in WO 99/08729 (p.11), albeit a polyacrylic acid-based coating is used therein.

As further non-limiting examples of stents as well as guidewires and angioplasty balloons which are suitable in the practising of the present invention, mention can be made of those disclosed in "*Interventional Vascular Product Guide*". Ed.: Leon M.B., Mintz G.S., Publ. Martin Dunitz, 1999. ,

It deserves to be mentioned that stents as well as guidewires coated with phosphorylcholine are commercially available. A typical such stent is Biodivysion™ Phosphorylcholine (Biocompatibles Ltd., UK). Hence, coating of a medical device with phosphorylcholine having a biologically active agent dissolved or dispersed therein is readily accomplished by a person skilled in the art. A nonreleasing heparan-sulphate biocompatible coated stent is also commercially available.

In short, the general potency of the present medical device is based primarily on the following principles. Firstly, upon insertion of the medical device into a body, a rapid release of the biologically active agent is provided. More specifically, the outer second layer will normally release at least 50% of its biologically active component within 1-24 h after insertion, thereby alleviating acute disorders. Secondly, the inner first layer will thereafter provide a sustained release (*vide supra*) of its biologically active component, thereby providing a long-term therapeutic effect as well as a prophylactic effect. This combined "pulsed" effect of the two layers provides a versatile treatment regimen.

Although the examples above disclose the preparation of coated stents only, it should be realised that these procedures are also readily adaptable for use on virtually any medical device, albeit particularly those specified *supra*. Hence, the features of the present medical device and method for use thereof are applicable within the field of medicine in general.

## Claims

1. Medical device chosen from sutures, surgical staples, implants and catheters adapted for insertion into a human or animal body, **characterised in that** its exterior surface is coated with
i) an inner first layer of a biocompatible carrier providing sustained release of a biologically active agent dissolved or dispersed therein;
*ii)* an outer second layer consisting of a film of said biologically active agent applied on said inner first layer, where said film optionally may contain at least one non-polymeric adjuvant, diluent or carrier.

2. Medical device according to claim 1, wherein said biocompatible carrier is a polymer.

3. Medical device according to claim 2, wherein said polymer is selected from polyamine-dextran-sulphate, poly fatty acid esters and polyurethane.

4. Medical device according to claim 3, wherein said polyamine-dextran-sulphate, poly fatty acid ester or polyurethane has an average molecular weight in the range of from 5 kDa to 100 kDa.

5. Medical device according to claim 4, wherein said poly fatty acid ester is polylactic acid (PLA), polyglycolic acid (PGA) or a copolymer of lactic acid and glycolic acid (PLGA).

6. Medical device according to any one of claims 1-5, wherein said non-polymeric adjuvant, diluent or carrier is selected from phosphorylcholine and derivatised phosphorylcholine, albumins, liposomes, and contrast medium; preferably iohexole.

7. Medical device according to any one of claims 1-6, wherein said biologically active agent is present in said inner first layer at a concentration of from 0.01 to 99 percent by weight and outer film layer.

8. Medical device according to any one of claims 1-7, wherein said inner first layer has a thickness in the range of from 0,5 to 1000 µm.

9. Medical device according to any one of claims 1-8, wherein said biologically active agent is a compound capable of providing release of nitric oxide.

10. Medical device according to claim 9, wherein said compound is a diethylenetriamine/nitric oxide adduct or sydnonimine, preferably molsidomine or linsidomine.

11. Medical device according to any one of claims 1-10, wherein said exterior surface consists of metal or a biocompatible organic or inorganic polymer.

12. Medical device according to claim 11, wherein said metal is selected from gold, silver, platinum, stainless steel, titanium and biocompatible alloys thereof.

13. Medical device according to claim 11, wherein said biocompatible organic or inorganic polymer is selected from fibrin, polytetrafluoroethylene (PTFE), silicone, silicone rubber, nylon and polyethylene perthalate (Dacron).
